# EUROPEAN PATENT APPLICATION

(11) **EP 4 623 902 A1**
(43) Date of publication of application: **01.10.2025**
(21) Application number: 23895130.5
(22) Date of filing: 24.11.2023
(51) Int. Cl.: A61K 9/08, A61K 38/28, A61K 47/18, A61P 3/10

(54) **COMPOSITION OF FAST-ACTING INSULIN (VARIANTS)**

(30) Priority: 26.11.2022 RU 2022130796
(71) Applicant: OOO "Geropharm", Sankt-Peterburg, 191119 (RU)
(72) Inventor: SHITIKOVA, Viktoriya Olegovna, Sankt- Peterburg, 190005 (RU); YUDAEVA, Nina Valeryevna, Sosnovyy Bor, 188540 (RU)
(74) Representative: Koitel, Raivo
(86) International application number: PCT/RU2023/050272
(87) International publication number: WO 2024/112231

(57) **Abstract**

The present invention relates to the field of pharmaceutical science and medicine, *i.e.,* to the compositions of fast-acting insulin preparations. More specifically, the present invention relates to the compositions comprising a fast-acting insulin and an amino acid as a stabilizer, where the amino acid may be selected from lysine and/or salt thereof, or a combination of lysine and/or salt thereof with arginine and/or salt thereof. Compositions of the invention exhibit better chemical and physical stability compared to the closest prior art.

## Description

### Field of the invention

The present invention relates to the field of pharmaceutical science and medicine, *i.e.,* to the compositions of fast-acting insulin preparations. More specifically, the present invention relates to compositions comprising a fast-acting insulin and an amino acid as a stabilizer, where the amino acid may be selected from lysine and/or salt thereof, or a combination of lysine and/or salt thereof with arginine and/or salt thereof. Compositions of the invention exhibit better chemical and physical stability compared to the closest prior art.

### Background of the invention

Insulin is a peptide hormone regulating blood glucose levels in mammals by initiating a signaling cascade that accelerates glucose uptake and glycogen production upon binding to the insulin receptor. When insulin production is compromised, glucose concentration in blood increases (chronic hyperglycemia), which is the primary diagnostic marker for type 1 diabetes (T1DM). However, in cases where signaling through insulin receptors is impaired, type 2 diabetes (T2DM) develops even when there is enough insulin hormone produced, which is a result of decreased tissue susceptibility to the action of insulin (insulin resistance). The efficiency of insulin in the treatment of diabetes due to the ability of this hormone to reduce blood glucose levels has been proven over decades of medical use. However, the demand for novel approaches to the treatment of diabetes mellitus is still in place due to increasing numbers of the disease and the need of considering individual patients' features and preferences [1].

Development of fast-acting and superfast-acting insulin analogues, such as insulin aspart (NovoRapid^{®}), insulin glulisine (Apidra^{®}) and insulin lispro (Humalog^{®}), having a faster onset of action and shorter duration of action, has become a significant advance in the treatment of type 1 and type 2 diabetes. Formulations of the drug preparations NovoRapid^{®}, Apidra^{®}, Humalog^{®} are the closest prior art in relation to compositions of the invention.

In a neutral solution, at pharmaceutical concentrations, insulin and insulin analogues occur in the form of stabilized zinc-containing hexamers consisting of three identical dimeric units. The delay in insulin action is largely due to the time it takes for the hexamers to dissociate into monomers and dimers which are capable of being absorbed [2,3]. The first class of fast-acting insulins (*e.g*., insulin lispro and aspart) was developed based on amino acid mutations in the human insulin sequence resulting in a weakened self-association of insulin oligomers. For example, replacing proline amino acid at position B28 with aspartic acid in insulin aspart reduces the molecules' tendency to form hexamers which is observed in a solution of soluble human insulin. Correspondingly, insulin aspart is absorbed much more quickly from subcutaneous adipose tissue compared to soluble human insulin, and is widely used for postprandial blood glucose control in diabetes mellitus patients [4]. The molecular structure of insulin lispro is identical to that of human insulin except for positions 28 and 29 in the B-chain of the molecule, where lysine and proline are arranged in reverse order (human insulin: B28ProB29Lys, lispro: B28LysB29Pro). The reverse arrangement of lysine and proline allows the lispro molecule to dissociate 2 times faster. In the insulin glulisine structure (3BLys29BGlu), the asparagine amino acid residue in position 3 of the human insulin molecule B-chain is replaced by lysine, and lysine residue in position 29 of the B-chain is replaced by glutamine, which contributes to stability of said drug preparation in solution in monomeric and dimeric form [1].

The chemical and physical stability of insulin preparations is essential to ensure their therapeutic effect and the absence of adverse reactions; in this respect, stability of the preparations must be maintained both during shelf-life and during use. Insulin compositions are manufactured, stored and used in vials, which requires of them to retain stability throughout shelf life. Stable insulin compositions are particularly desirable for use in delivery devices where they are exposed to elevated temperatures close to body temperature, and/or to mechanical stress, such as, *e.g.,* injection pens and continuous infusion systems (pump-assisted).

Fast-acting insulin analogues in monomeric or dimeric form are known to have reduced physicochemical stability compared to hexamers [5], *e.g.,* they form high molecular weight impurities; they also strongly tend to form aggregates (fibrillation), which manifests as turbidity and precipitate. High molecular weight impurities (dimers, trimers, and polymers) and aggregates cut down the administered dose of insulin, and can as well encourage irritation or immune reactions in a patient. Moreover, insoluble aggregates can clog and damage needles, cannulas, and pump tubing in continuous infusion systems. To ensure the quality of insulin compositions, it is necessary to enhance their chemical and physical stability.

Insulin aspart drug preparations are commercially available (for example, NovoRapid^{®} (Novo Nordisk A/S), RinFast^{®} (GEROPHARM OOO). It has been shown that overall glycemic control can be improved by adding the excipient nicotinamide, which is able to accelerate absorption of insulin aspart compared to insulin aspart preparations lacking nicotinamide. Specifically, Fiasp^{®} (comprising nicotinamide) was shown to provide improved overall glycemic control and better postprandial glycemic control without increasing overall risk of severe or confirmed hypoglycemia in T1DM and T2DM patients as compared to NovoRapid^{®} (lacking nicotinamide). Pharmacokinetics of the fast-acting insulin aspart Fiasp^{®} may better mimic the rapid endogenous secretion of prandial insulin and thus provide improved postprandial glycemic control compared with insulin aspart [6].

Nicotinamide can accelerate insulin absorption (WO 91/09617), including insulin aspart (WO/9610417), however, it has a negative effect on chemical stability, increasing the content of impurities (RU2533217). The chemical stability of the Fiasp^{®} drug preparation (DP) formulation is enhanced by introduction of arginine, which can be seen as a decrease in dimeric and polymeric content and deamidated insulin content during storage (RU2533217). FIASP^{®} DP formulation is another closest prior art of the present invention.

Patent RU2533217 discloses (see example 2) that arginine, added to the compositions containing insulin aspart and nicotinamide, reduces amounts of forming degradation products, especially amounts of high molecular weight proteins and deamidated forms, however, at the same time it reduces physical stability, measured as lag time in the thioflavin T assay, and the physical stability continues to decline even more as the arginine concentration increases.

A report from a small scale (n = 37) 6-week study of the infusion systems' susceptibility to clogging and malfunction reveals no observed cases of infusion-system occlusions with either FIASP DP (25 subjects) or insulin aspart (12 subjects). However, unexplained hyperglycemia and early replacement of infusion sets were more common with FIASP compared to insulin aspart [7].

Therefore, there remains a current need for compositions of fast-acting insulins featuring enhanced chemical stability and, at the same time, acceptable physical stability. The technical problem outlined above is especially relevant for the fast-acting insulin compositions, such as, e.g., insulin aspart, insulin lispro, or insulin glulisine, characterized by lower stability compared to hexameric forms of insulin, and for the compositions comprising nicotinamide, which, while able to accelerate insulin absorption, negatively affects the insulin chemical stability.

### SUMMARY OF THE INVENTION

The inventors have unexpectedly discovered that compositions of fast-acting insulin containing an amino acid as a stabilizer, said amino acid being lysine and/or a salt thereof, or a combination of lysine and/or a salt thereof with arginine and/or a salt thereof, have improved chemical and physical stability compared to insulin compositions without added amino acids, as well as compared to insulin compositions containing arginine amino acid as a stabilizer.

Accordingly, the present invention relates to the compositions with improved chemical and physical stability comprising a fast-acting insulin and an amino acid, said amino acid being lysine or a salt thereof, or a combination of lysine or a salt thereof with arginine or a salt thereof.

The present invention relates to compositions for reducing blood glucose levels in a subject, the compositions comprising a fast-acting insulin and an amino acid, said amino acid being lysine or a salt thereof, or a combination of lysine or a salt thereof with arginine or a salt thereof.

In preferred embodiments, the insulin is insulin aspart, insulin lispro, or insulin glulisine.

Insulin glulisine compositions optionally comprise methionine.

The fast-acting insulin concentration in the compositions of the invention ranges from about 0.2 mM to about 2.0 mM (from about 33 U/ml to about 333 U/ml), preferably from about 0.3 mM to about 1.2 mM (from about 50 U/ml to about 200 U/ml), and even more preferably is 0.6 mM (100 U/ml).

In one embodiment of the invention, the compositions have a pH from 6.5 to 8.5. In yet another embodiment, the compositions have a pH of from 6.6 to 7.4. In one embodiment, the pH is 7, 7.3, 7.4, or 7.5.

Compositions of the invention, comprising insulin aspart, optionally comprise a nicotinic acid compound selected from nicotinamide (niacinamide), nicotinic acid (niacin), and/or a salt and/or any combination thereof; preferably, the nicotinic acid compound is nicotinamide. The concentration of nicotinamide or other nicotinic acid compound in the compositions of the invention comprising insulin aspart ranges from about 1 mM to about 200 mM.

The concentration of lysine and/or a salt thereof, or the total concentration of lysine and/or a salt thereof in combination with arginine and/or a salt thereof ranges from about 1 mM to about 100 mM.

In compositions comprising a combination of lysine and/or a salt thereof and arginine and/or a salt thereof, the molar ratio of the amino acids is from 4000:1 to 1:4000, respectively, said range including each and every integer ratio, *e.g.,* 100: 1, 99:1, 98:1, *etc.* In one preferred embodiment, the lysine: arginine molar ratio is 1:1, 4:5, 5:4, 6:4; 5:2, or 2:5.

The compositions of the invention may further comprise protease inhibitor(s), metal ions, buffer systems, pH adjusting agents, preserving agent(s), isotonic agent(s), chelating agent(s), stabilizers and surfactants.

In one embodiment, the compositions of the invention are aqueous compositions, i.e., compositions that comprise water and occur as solutions or suspensions.

The compositions of the present invention can be used for treating or preventing hyperglycemia, type 2 diabetes mellitus, impaired glucose tolerance, and type 1 diabetes mellitus.

In one preferred embodiment, the compositions of the invention are administered parenterally. Parenteral administration can be performed by subcutaneous, intramuscular, intraperitoneal, or intravenous injection using a syringe, which is optionally an injection pen. Alternatively, parenteral administration can be performed using an infusion pump.

Accordingly, the present invention also relates to injectable insulin preparations comprising compositions of the present invention.

### TERMS AND DEFINITIONS

As used herein, the term "human insulin" means a human hormone having well-known structure and properties. Insulin is a 51 amino acid polypeptide divided up into 2 amino acid chains: A-chain, consisting of 21 amino acids, and B-chain, consisting of 30 amino acids. The chains are interconnected with 2 disulfide bridges. For many years, insulin drug preparations have been used to treat diabetes mellitus, and recently not only insulins of natural origin, but also insulin derivatives and analogues come into use.

As used herein, the term "insulin analogue" means a polypeptide derived from the primary structure of naturally occurring insulin, such as human insulin, by mutation. One or more mutations are obtained by deletion and/or substitution of at least one amino acid residue occurring in the natural insulin and/or by addition of at least one amino acid residue thereto. Mutations in the insulin molecule are indicated by the respective chain reference (A or B), position, and a three-letter code for the amino acid replacing the native one.

The term "fast-acting insulin" or "short-acting insulin" refers to the insulin analogues and/or insulin derivatives that start to act within 5-15 minutes and have a duration of action of 3-4 hours. Examples of fast-acting insulins include, but are not limited to the following: insulin aspart, insulin lispro and insulin glulisine.

The terms "monomeric human insulin analogue" and "monomeric insulin analogue" are well known in the art and usually refer to the fast-acting human insulin analogues. They include, for example, insulin lispro (Humalog^{®}), insulin aspart (NovoRapid^{®}), and insulin glulisine (Apidra^{®}).

The content of fast-acting insulin in compositions of the invention may range between about 0.2 mM and about 2.0 mM (between about 33 and about 333 international units (IU/ml), preferably ranges between about 0.3 mM and about 1.2 mM (between about 50 and about 200 U/ml), most preferably is 0.6 mM (100 U/ml), in injectable preparations. However, the insulin compound content may be higher for parenteral administration performed with other purposes.

In this context, the IU unit corresponds to 6 nmol.

As used herein, the term "fast-acting insulin composition" means a product comprising a monomeric insulin analogue and an amino acid selected from lysine and/or a salt thereof, or a combination of lysine and/or a salt thereof with arginine and/or a salt thereof. Preferably, the monomer insulin analogue in the composition is insulin aspart, insulin lispro, or insulin glulisine. The insulin aspart composition of the present invention may optionally comprise a nicotinic acid compound selected from nicotinamide (niacinamide), nicotinic acid (niacin), and/or a salt and/or any combination thereof. The fast-acting insulin composition may optionally further comprise other excipients, such as preservatives, chelating agents, isotonic agents, filling agents, stabilizers, antioxidants, polymers and surfactants, metal ions, oil carriers and proteins (e.g., human serum albumin, gelatin, or proteins), pH adjusting agents, wherein the fast-acting insulin composition can be used to treat, prevent, or reduce the severity of a disease or disorder by administering said insulin preparation to a human. Thus, the fast-acting insulin composition of the invention can be designated as a "pharmaceutical composition".

The terms "protein preparation", "protein composition", and "protein" can be used interchangeably herein meaning any drug product comprising, as an active substance, a molecule comprised of the polypeptide chains consisting of amino acid residues linked linearly by peptide bonds, independently or in combination with other compounds. Insulin composition or insulin, for example, are particular cases and are encompassed by these terms.

The terms "lysine" and "arginine" refer to amino acids and include D- and L-enantiomers, as well as mixtures thereof. The term also includes any pharmacologically acceptable salts of lysine and arginine. Lysine and arginine readily form salts, such as hydrochlorides.

The term "a nicotinic acid compound" includes nicotinamide (niacinamide), nicotinic acid (niacin), and/or salts and/or any combination thereof.

The buffer may be selected from the group including but not limited to sodium acetate, sodium carbonate, citrate, sodium dihydrogen phosphate, sodium hydrogen phosphate, sodium phosphate, as well as tris(hydroxymethyl)aminomethane, bicine, tricine, malic acid, succinate, maleic acid, fumaric acid, tartaric acid, aspartic acid, or mixtures thereof. Each of these specific buffers, and each of combinations thereof is an alternative embodiment of the invention.

The fast-acting insulin composition of the invention may further comprise other ingredients normally used in insulin preparations, *e.g.,* zinc complexing agents.

Isotonic agents, such as glycerol, mannitol, sodium chloride, dextrose, and the like, may also be present in the compositions of the invention. An isotonic agent is a physiologically acceptable compound that imparts suitable tonicity to a composition, preventing diffusion of water across cell membranes in contact with a pharmaceutical composition.

The composition of the invention may comprise a pharmaceutically acceptable preserving agent. The preserving agent present in the composition of the invention may be phenol, m-cresol, methylparaben, *etc.*

The composition of the present invention may further comprise a chelating agent. The use of chelating agents in pharmaceutical preparations is well known to those skilled in the art.

The composition of the invention may further comprise a stabilizer. As used herein, the term "stabilizer" refers to the chemicals added to pharmaceutical preparations comprising a polypeptide for stabilizing said polypeptide, i.e. for extending the shelf life and/or usage period of such preparations.

As used herein, the term "surfactant" (surface-active compound, SAC) refers to any molecules or ions that consist of a water-soluble (hydrophilic) portion, a head portion, and a fat-soluble (lipophilic) segment. SACs concentrate predominantly at the interface, with the hydrophilic part directed towards water (hydrophilic phase), and the lipophilic part directed towards oil or hydrophobic phase (i.e. glass, air, oil, etc.). SACs reduce the surface tension of liquids. They are also known as amphipathic compounds. The term "detergent" is generally synonymous with the term "surfactant". The use of SACs in pharmaceutical preparations is well known to those skilled in the art. Such SACs include, for example, polysorbate 20, polysorbate 80, poloxamer 188, and the like; these substances are described in the current Pharmacopoeias (USP, EP) as excipients.

As used herein, the term "stability" refers to the chemical and physical stability of compositions with monomeric insulin analogues.

As used herein, the term "chemical stability" in relation to a protein composition (protein preparation) and/or a protein refers to changing the covalent structure of the protein resulting in formation of chemical degradation products with potentially lower biological activity and/or potentially enhanced immunogenic properties compared to native protein structure. Various chemical degradation products may form depending on the type and nature of the native protein, and on environmental factors the protein is exposed to. An increase in chemical degradation products is commonly observed during storage and use of a protein preparation. Most proteins are prone to deamidation; other degradation pathways involve formation of high molecular weight products, where two or more protein molecules covalently bind to each other via transamidation and/or disulfide bonds, resulting in the covalently linked dimeric, oligomeric, and polymeric degradation products [8]. Another chemical degradation variant worth mentioning is oxidation (e.g., oxidation of methionine residues). The chemical stability of a protein preparation and/or a protein can be assessed by measuring the amount of chemical degradation products at different time points after exposure to various environmental conditions (formation of degradation products can often be accelerated, for example, by increasing the temperature). The amount of each individual degradation product is commonly determined by separating the degradation products based on their molecular size and/or charge using various chromatography techniques (e.g., size exclusion liquid chromatography (SEC-HPLC), and/or reverse-phase high-performance liquid chromatography (RP-HPLC)). Since the high molecular weight protein products are potentially immunogenic and biologically inactive, the levels of the high molecular weight proteins should remain low.

Physical instability of a protein composition (protein preparation) and/or a protein may result from aggregation of the protein molecules forming higher order polymers or even precipitates. Physical denaturation of insulin is known as fibrillation. In the fibrillar state, elongated peptide chains are arranged in parallel or antiparallel orientation and are interconnected via hydrogen bonds, forming the so-called β-structure or β-sheets. Fibrils typically represent the lowest energy state of a protein; regeneration of the protein from this state to the native properly folded state is only possible in a strong alkaline environment. Insulin fibrils take the form of gels or precipitates. Exposure to thermomechanical stress and/or interaction with interphase boundaries and hydrophobic surfaces are factors promoting acceleration of fibril formation. Fibrillation is believed to occur due to insulin monomerization. Monomeric insulin analogues, which readily dissociate from the hexameric unit to the monomeric form, are more likely to form fibrils compared to human insulin.

Physical stability can be assessed by methods well known in the art, for example, by measuring turbidity (optical density). Turbidity results from aggregation or precipitation of proteins or complexes in compositions.

The physical stability of aqueous protein preparations can also be assessed by using a spectroscopic agent or probe of the conformational status of the protein. The probe is preferably a small molecule that predominantly binds to non-native protein conformers. One example of a small molecule probe for spectroscopic protein structure determination is thioflavin T. Thioflavin T is a fluorescent dye widely used for detection of amyloid fibrils. In the presence of fibrils and possibly some other protein configurations thioflavin T, upon binding to the fibrillar form of the protein, produces a new excitation maximum at approximately 445 nm, and enhances emission at approximately 485 nm. Unbound thioflavin T is substantively non-fluorescent at the above wavelengths.

A "stable composition" is the one wherein the extent of protein aggregation and impurities content fall within normal limits and do not exceed these limits over time.

The term "aqueous preparation" refers to a preparation containing water. The terms "aqueous solution" and "aqueous suspension" refer to a solution or suspension containing water, respectively. Aqueous suspensions may comprise active compounds in admixture with excipients suitable for preparing the aqueous suspensions.

### EXAMPLES

### Example 1. Preparing compositions of the invention which comprise insulin aspart

In one embodiment, the pharmaceutical compositions of the invention are prepared as aqueous solutions. Table 1 shows the qualitative and quantitative content of some of the prepared compositions. The compositions of the invention include, but are not limited to compositions 4-17 listed in Table 1. Composition 1 formulation corresponds to one of the closest prior art compositions, i.e., Fiasp^{®} DP. Composition 2 formulation is similar to Composition 1 except that the former lacks L-arginine hydrochloride. Composition 3 formulation is the same as Composition 1 except that the former lacks nicotinamide. Composition 4 formulation is similar to Composition 3 except that the former comprises added L-lysine hydrochloride in place of L-arginine hydrochloride. Composition 5 formulation is similar to Composition 3 except that the former comprises added L-lysine and L-arginine combination.

The method for producing the compositions includes:
a) preparing an insulin aspart solution by dissolving the insulin aspart in water or a buffer;
b) preparing a zinc salt solution by dissolving the zinc salt in water or a buffer;
c) preparing a solution of preserving agents (phenolic compounds) by dissolving them in water or a buffer;
d) preparing an isotonic agent solution by dissolving the isotonic agent in water or a buffer;
e) preparing a nicotinamide solution and/or a solution of other nicotinic acid compound (if present in the composition) by dissolving the same in water or a buffer;
f) preparing a solution of lysine and/or arginine by dissolving the amino acids and/or salts thereof in water or a buffer;
g) mixing Solution a), Solutions b), c), d), e) if present, and Solution f), in accordance with the composition formulations given in Table 1;
h) adjusting pH value of the mixture g) to 6.6-7.4, followed by sterilizing filtration.
i) aseptic filling the prepared compositions into cartridges.

Compositions of the invention can be produced using any other method.

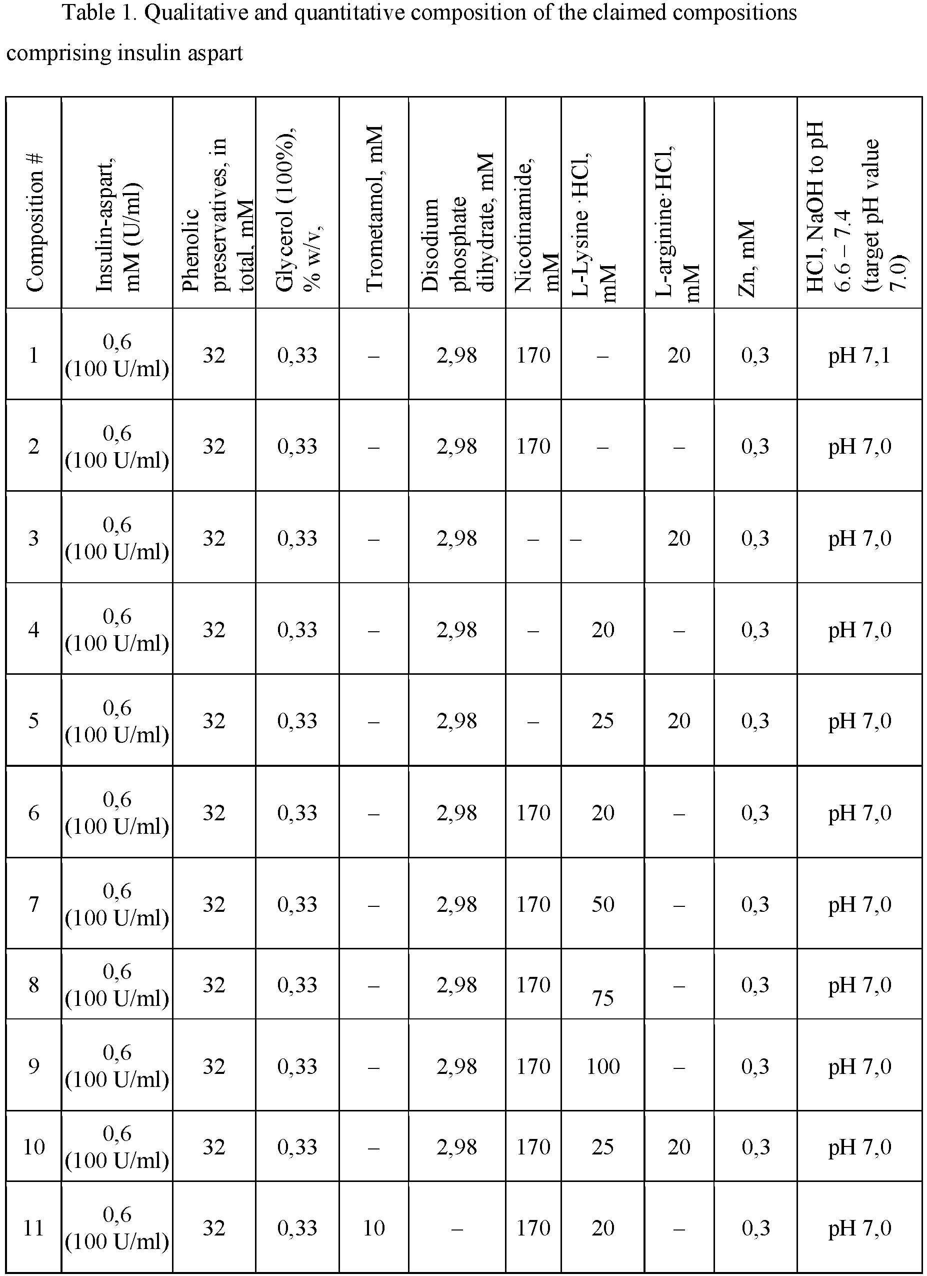

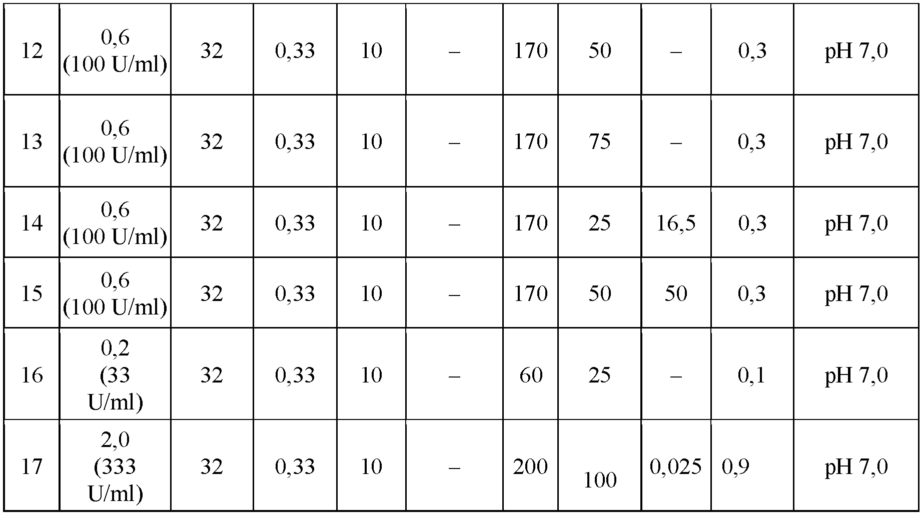

### Example 2. Analysis of physical and chemical stability of the claimed compositions comprising insulin aspart

Chemical stability was determined for compositions 1-17 produced according to example 1, after thermostatting the cartridges aseptically filled with the compositions at 40° C over the course of 1 and 2 weeks.

Insulin aspart-related impurities were determined using HPLC with UV detection in a column packed with octadecyl silica gel having 5 µm particle size and 300 Å pore size, with a mobile phase flow rate of 1.0 ml/min, at 214 nm wavelength.

Elution was carried out in a gradient mode using mobile phase comprised of the following components:
*mobile phase A (MP A):* acetonitrile:buffer solution, pH 3,4 (1,4 % solution (w/V) of sodium sulfate anhydrous, 0,13 % solution (V/V) of concentrated orthophosphoric acid, 2 M sodium hydroxide to pH 3,4 ± 0,05) 10:90 (V/V);
*mobile phase B (MP B):* acetonitrile:water 50:50 (V/V).

The amount of B28IsoAsp, deamidated forms (B3iso, A21Asp, B3Asp) and other related impurities was determined using internal normalization method by the absorption area of the corresponding peaks measured as a % of the peak area sum for the peaks which eluted after nicotinamide and preservatives.

The amount of high molecular weight protein (HMWP) was determined by size exclusion liquid chromatography in a column packed with 10 µm particle size, 125 Å pore diameter hydrophilic chromatography silica gel. A mixture of acetonitrile: glacial acetic acid: 0.1% arginine solution (20:15:65) (V/V/V) was used as an eluent. Elution was carried out in isocratic mode with a flow rate of 0.5 ml/min at a detection wavelength of 276 nm.

The HMWP content was determined using intemal normalization method, by the sum of absorption areas for all peaks having shorter retention times compared to the insulin aspart monomer peak. Peaks having longer retention times than that of the insulin aspart monomer (*i.e.,* nicotinamide and preservatives) were excluded from the calculation.

The physical stability of the protein compositions was evaluated by testing the tendency for fibrillation in the Thioflavin-T (ThT) assay. The chosen method is described in the literature, e.g., in RU2533217. The tendency for forming fibrils was determined for compositions 1, 3-17 in example 1, and for the finished pharmaceutical product of the original Fiasp DP. Composition 1 was similar to the Fiasp DP formulation. The compositions were assayed immediately after preparation.

### Preparation of the solutions

### Thioflavin stock solution

A precise portion of the dry Thioflavin T reagent is dissolved in methanol to the concentration of 1 mg/ml (corresponding to 3.14 mM). The solution is thoroughly vortexed and stored protected from light at + 2 - 8 °C for up to 6 months.

### Thioflavin working solution

A working 200 µM thioflavin solution in purified water is prepared. The volume ratio of the stock solution to purified water is adjusted to 1:14.7.

The solution is used freshly prepared.

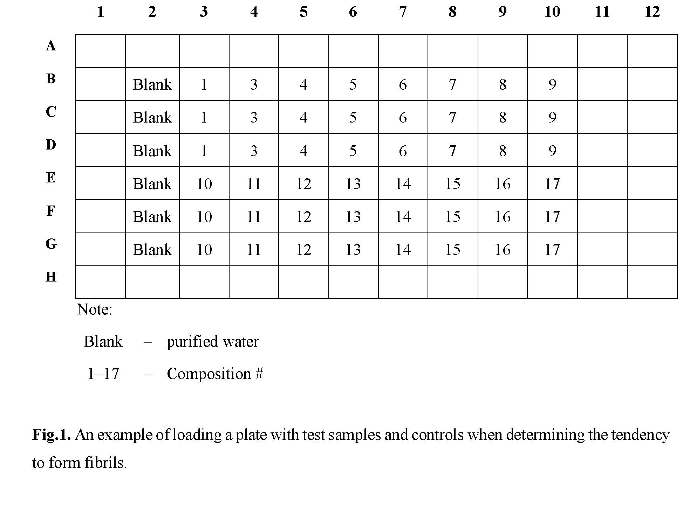

100 µl sample aliquots in triplicates are placed in a black 96-well plate, and purified water is used as a control sample. 10 µl of the thioflavin working solution was added to all wells. The edge wells are not used when filling the plate (see Fig. 1). The plate is sealed with transparent film and placed in CLARIOstar multi-mode plate reader (BMG, Germany). The fluorescent signal is detected every 20 minutes for 12 hours at a wavelength of 445/485 nm. Between readings, the plate is stirred at 700 rpm. Stirring and detection are performed at 37 °C.

Based on the measured results, the signal in relative fluorescence units is plotted against time in hours for each of the samples. A 4-parameter model is used for analysis.

The graph is used to determine T1/2max, i.e., the time required for half of the analyzed sample to aggregate. When a graph takes the form of a straight line at zero value level, the sample is considered to be not prone to aggregation; in other cases, tendency to aggregation is measured by T1/2max.

Along with T1/2max, the ThT fluorescence curve is used to visually determine the lag time as the time point where ThT fluorescence is different from the background level.

Table 2 shows the results of the chemical and physical stability studies for Compositions 1, 3-17 comprising insulin aspart.

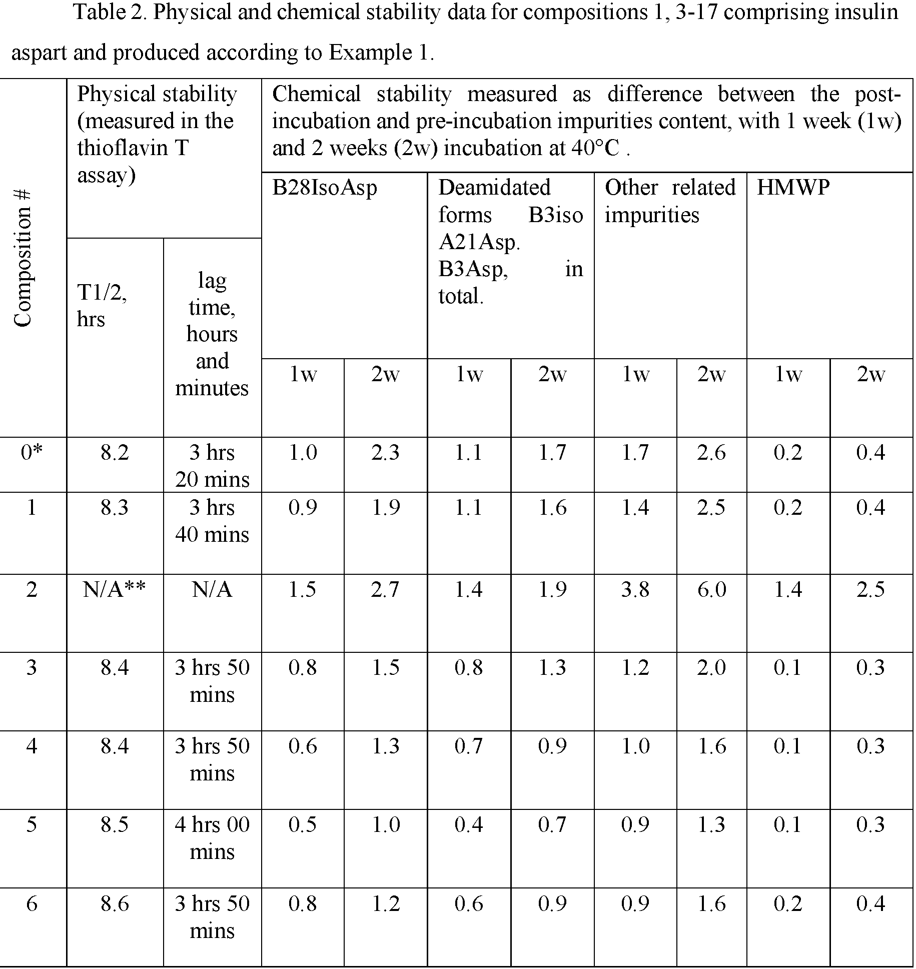

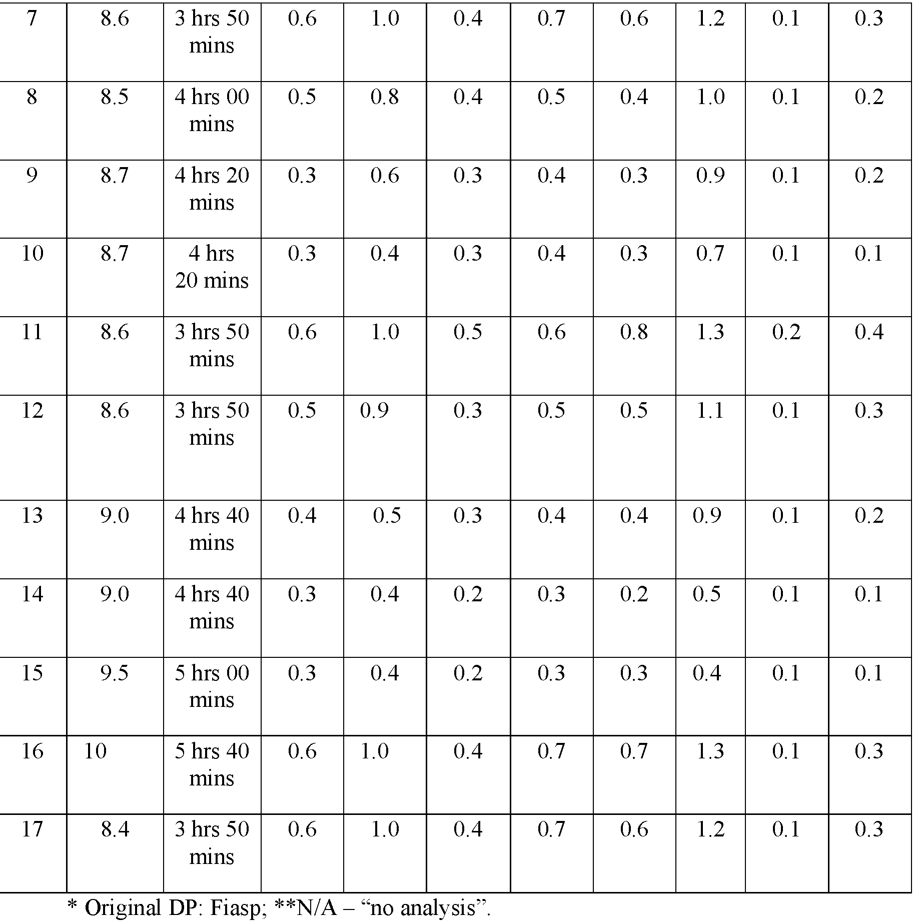

Based on the chemical and physical stability studies for the insulin aspart compositions prepared with phosphate buffer or Tris-buffer, it was unexpectedly found that adding lysine and/or a salt thereof, and/or a combination of lysine and/or a salt thereof with arginine and/or a salt thereof to compositions of the invention results in significant and reliable decrease in impurities compared to the prototype after 2 weeks of storage at 40°C, while the physical stability is enhanced relative to that of the prototype.

Nicotinamide used in the composition without added amino acids (Composition 2) has a negative effect on chemical stability during storage. Arginine in Composition 1, which is similar to FIASP DP, prevents chemical degradation - said degradation resulting, in particular, from adding nicotinamide - which occurs as a decrease in HMWP and deamidated insulins after 2 weeks of storage at 40°C compared to the impurities content in Composition 2 lacking arginine. Replacing arginine in Composition 1 with the equimolar amount of lysine (Composition 6) reliably reduces impurities content; increasing lysine concentration to 100 mM promotes even greater reduction in impurities. Lysine concentrations greater than 100 mM are undesirable due to degrading physical stability of the compositions. The lowest amount of impurities was observed for the combination of lysine and arginine (Composition 10).

Similar results were obtained for compositions prepared using Tris-buffer (Compositions 11-17), with better chemical stability compared to their counterparts prepared using phosphate buffer.

A reliable advantage of stabilization by lysine or a combination of lysine and arginine over arginine stabilization was also shown for the compositions lacking nicotinamide (Compositions 3-5).

### Example 3. Preparation of the claimed compositions comprising insulin glulisine

In one embodiment, the pharmaceutical compositions of the invention are prepared as aqueous solutions. Table 3 shows the qualitative and quantitative content of some of the prepared compositions. The compositions of the invention include, but are not limited to compositions 2-7 listed in Table 3. Formulation of Composition 1 corresponds to the original Apidra^{®} DP, 100 U/ml subcutaneous solution. Compositions 2-7 are similar to the original DP except that they comprise added L-lysine hydrochloride or combinations of L-lysine hydrochloride and L-arginine hydrochloride.

The method for producing the compositions includes:
a) preparing an insulin glulisine solution by dissolving the insulin glulisine in water or a buffer;
b) preparing a preserving agent solution by dissolving the agent in water or a buffer;
c) preparing an isotonic agent solution by dissolving the isotonic agent in water or a buffer;
d) preparing a surfactant solution by dissolving the surfactant in water or a buffer;
e) preparing a solution of lysine and/or combination of lysine and arginine by dissolving the amino acids and/or salts thereof in water or a buffer;
f) mixing Solution a) and Solutions b), c), d), and e), in accordance with the composition formulations given in Table 3;
g) adjusting pH value of the mixture f) to 7.3, followed by sterilizing filtration.
h) aseptic filling the prepared compositions into cartridges.

Compositions of the invention can be obtained using any other means.

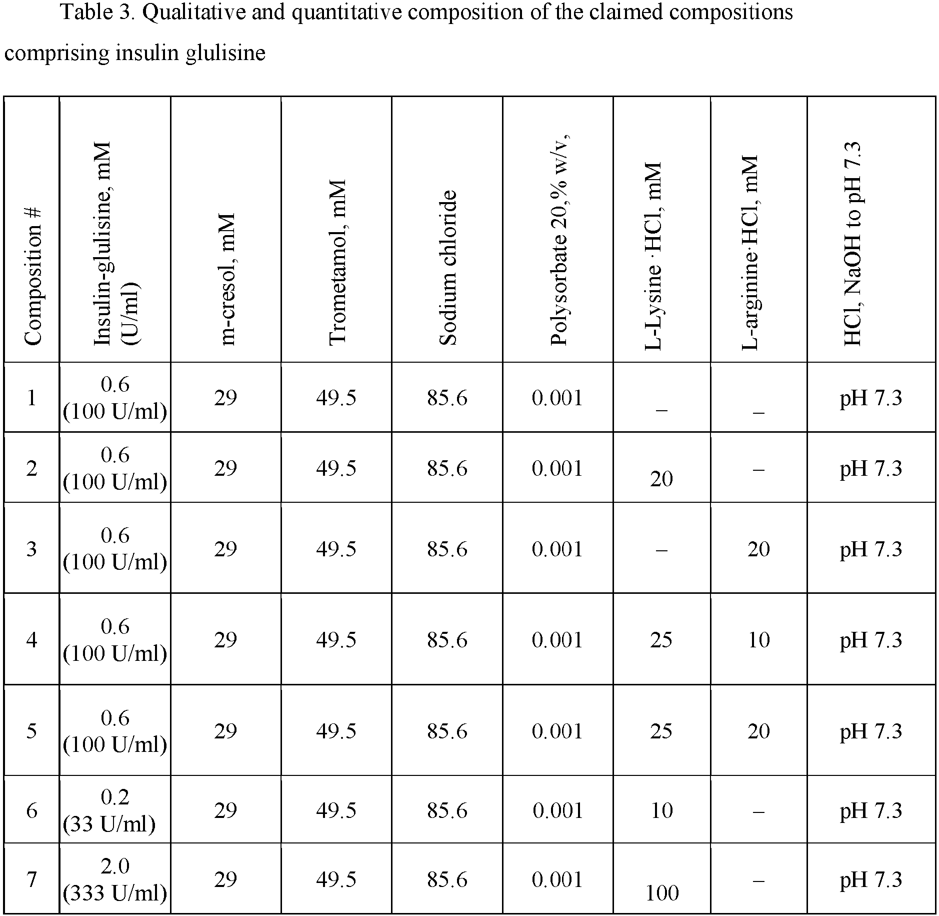

### Example 4. Analysis of physical and chemical stability of the claimed compositions comprising insulin glulisine

Chemical stability was determined for compositions 1-7, prepared as in Example 3, after thermostatting in aseptically filled cartridges at 40° C for 1 and 2 weeks.

Insulin glulisine-related impurities were determined using HPLC with UV detection in a column packed with octadecyl silica gel having 5 µm particle size and 100 Å pore size, with a mobile phase flow rate of 1.0 ml/min, at 214 nm wavelength.

Elution was carried out in a gradient mode using mobile phase comprised of the following components:
*mobile phase A (MP A):* acetonitrile:buffer solution, pH 2,3 (0,1 M sodium sulfate solution, orthophosphoric acid q.s. pH 2.3) 10:90 (V:V);
*mobile phase B (MP* 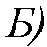 *:* acetonitrile:buffer solution, pH 2,3 (0,1 M sodium sulfate solution, orthophosphoric acid q.s. pH 2.3) 50:50 (V:V).

The amount of A21-desamidoinsulin glulisine and other related impurities was determined using intemal normalization method, by the sum of absorption areas of the corresponding peaks, measured as a percentage of the sum of areas for the peaks eluted after metacresol.

The amount of HMWP in compositions comprising insulin glulisine was determined using a method similar to HMWP determination in compositions comprising insulin aspart (see Example 2). The HMWP content was determined using intemal normalization method, by the sum of absorption areas for all peaks having shorter retention times compared to the insulin glulisine monomer peak. Peaks having longer retention times than that of the insulin glulisine monomer (metacresol peak) were excluded from the calculation.

The physical stability of the compositions comprising insulin glulisine was evaluated similar to the physical stability of the compositions comprising insulin aspart (see Example 2).

Table 4 shows the results of stability studies for compositions comprising insulin glulisine.

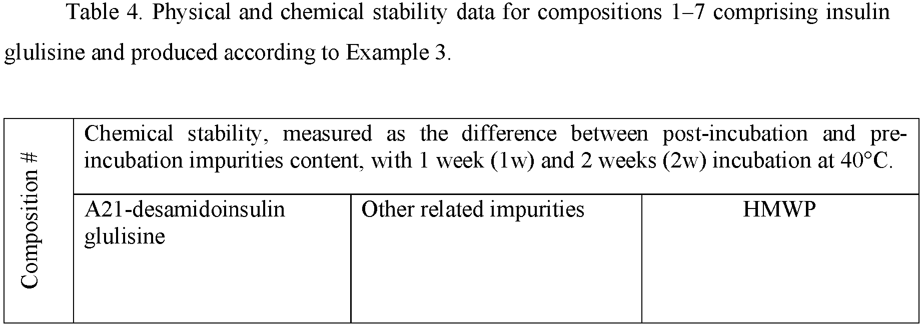

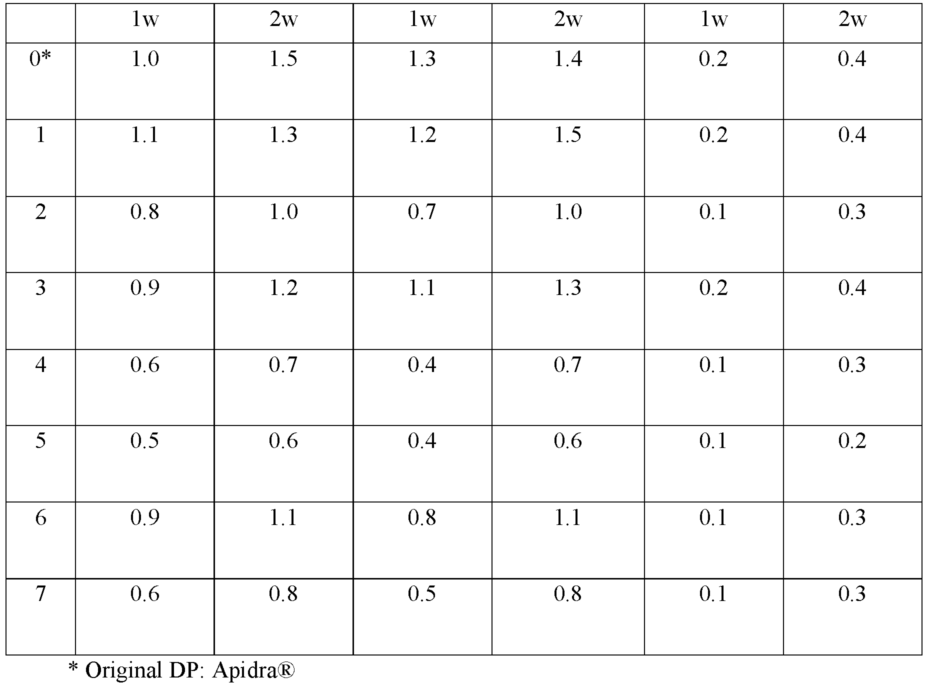

Based on the chemical and physical stability studies for the compositions 1-7 comprising insulin glulisine, it was unexpectedly found that adding lysine and/or a salt thereof, and/or a combination of lysine and/or a salt thereof with arginine and/or a salt thereof to compositions of the invention results in significant and reliable decrease in impurities compared to the prototype after 2 weeks of storage at 40°C, while the physical stability is enhanced relative to that of the prototype.

Increasing lysine concentration to 100 mM helps to reduce the amount of impurities. Lysine concentrations greater than 100 mM are undesirable due to degrading physical stability of the compositions. The lowest amount of impurities was observed for the combination of lysine and arginine.

### Example 5. Preparation of the claimed compositions comprising insulin lispro

In one embodiment, the pharmaceutical compositions of the invention are prepared as aqueous solutions. Table 5 shows the qualitative and quantitative content of some of the prepared compositions. Compositions of the invention include, but are not limited to

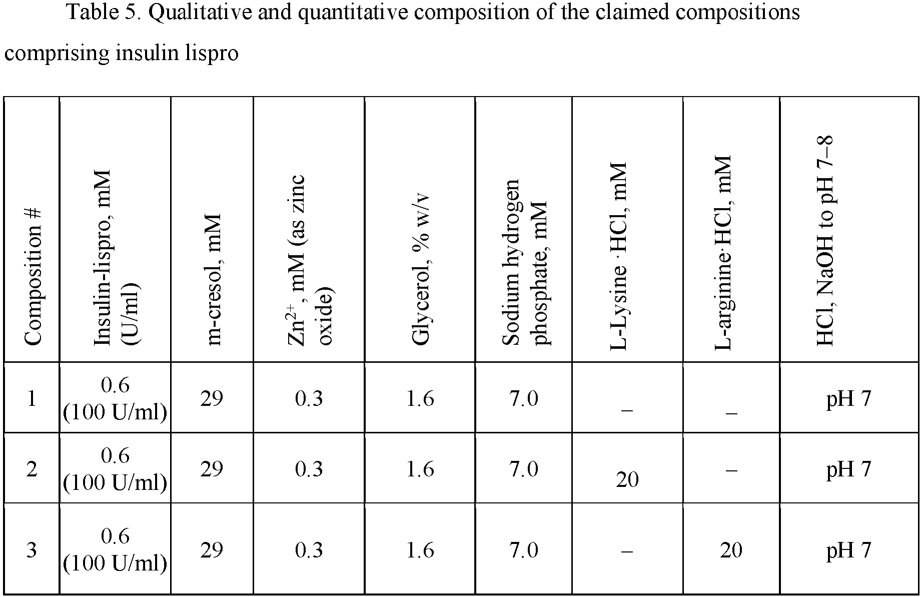

Compositions 2-8 listed in Table 5. Composition 1 formulation is similar to the original Humalog DP, 100 U/ml intravenous/subcutaneous solution. Compositions 2-7 are similar to the original DP formulation with addition of L-lysine hydrochloride or combinations of L-lysine hydrochloride and L-arginine hydrochloride.

The method for producing the compositions includes:
a) preparing an insulin solution by dissolving the insulin in water or a buffer;
b) preparing a zinc solution by dissolving zinc oxide in a solution of dilute hydrochloric acid;
c) preparing a preserving agent solution by dissolving the agent in water or a buffer;
d) preparing an isotonic agent solution by dissolving the agent in water or a buffer;
e) preparing a solution of lysine and/or arginine by dissolving the amino acids and/or salts thereof in water or a buffer;
f) mixing Solution a) and Solutions b), c), d), e) in accordance with the composition formulations given in Table 1;
g) adjusting pH value of the mixture e) to 7-8, followed by sterilizing filtration.
h) aseptic filling the prepared compositions into cartridges.

Compositions of the invention can be obtained using any other method.

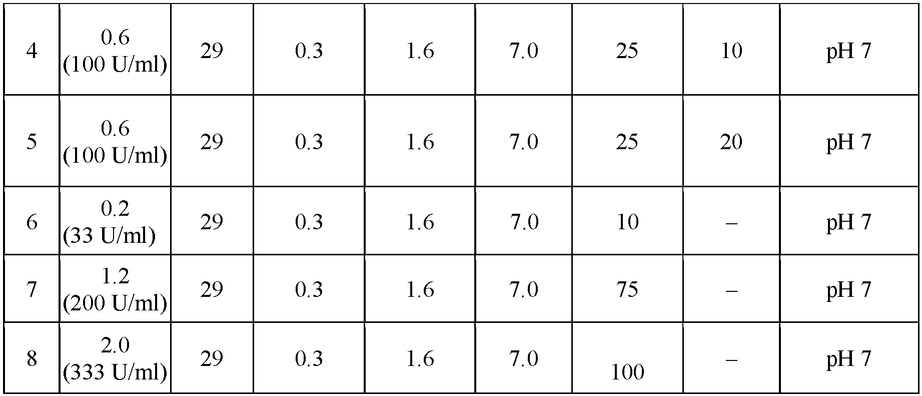

### Example 6. Analysis of physical and chemical stability of the claimed compositions comprising insulin lispro

Chemical stability was determined for compositions 1-8 (compositions comprising insulin lispro) prepared as in Example 5 after thermostatting in aseptically filled cartridges at 40° C for 1 and 2 weeks.

A21-desamidoinsulin lispro and other insulin lispro-related impurities were determined using HPLC with UV detection in a column packed with octadecyl silica gel having 3,5 µm particle size. A mobile phase consisting of the following components was used as an eluent:
*mobile phase A (MP A):* sodium perchlorate buffer solution pH 2,2;
*mobile phase B (MP B):* acetonitrile for chromatography.

Elution was carried out in a gradient mode with a mobile phase flow rate of 0,9 ml/min and detection wavelength of 214 nm.

The amount of A21-desamidoinsulin lispro and other related impurities was determined using intemal normalization method, by the sum of absorption areas of the corresponding peaks, measured as a percentage of the sum of areas for the peaks eluted after metacresol.

The amount of HMWP in compositions comprising insulin lispro was determined using a method similar to HMWP determination in compositions comprising insulin aspart or insulin glulisine (see Example 2). The HMWP content was determined using internal normalization method, by the sum of absorption areas for all peaks having shorter retention times compared to the insulin lispro monomer peak. Peaks having longer retention times than that of the insulin lispro monomer (metacresol peak) were excluded from the calculation.

The physical stability of the compositions comprising insulin lispro was evaluated similar to the physical stability of the compositions comprising insulin aspart (see Example 2).

Table 6 shows the results of the chemical and physical stability studies for compositions comprising insulin lispro.

Based on the chemical and physical stability studies for Compositions 1-8 comprising insulin lispro, it was unexpectedly found that adding lysine and/or a salt thereof, and/or a combination of lysine and/or a salt thereof with arginine and/or a salt thereof to compositions of the invention results in significant and reliable decrease in impurities compared to the prototype after 2 weeks of storage at 40°C, while the physical stability is enhanced relative to that of the prototype.

Increasing lysine concentration to 100 mM helps to reduce the amount of impurities. Lysine concentrations greater than 100 mM are undesirable due to degrading physical stability of the compositions. The lowest amount of impurities was observed for the combination of lysine and arginine.

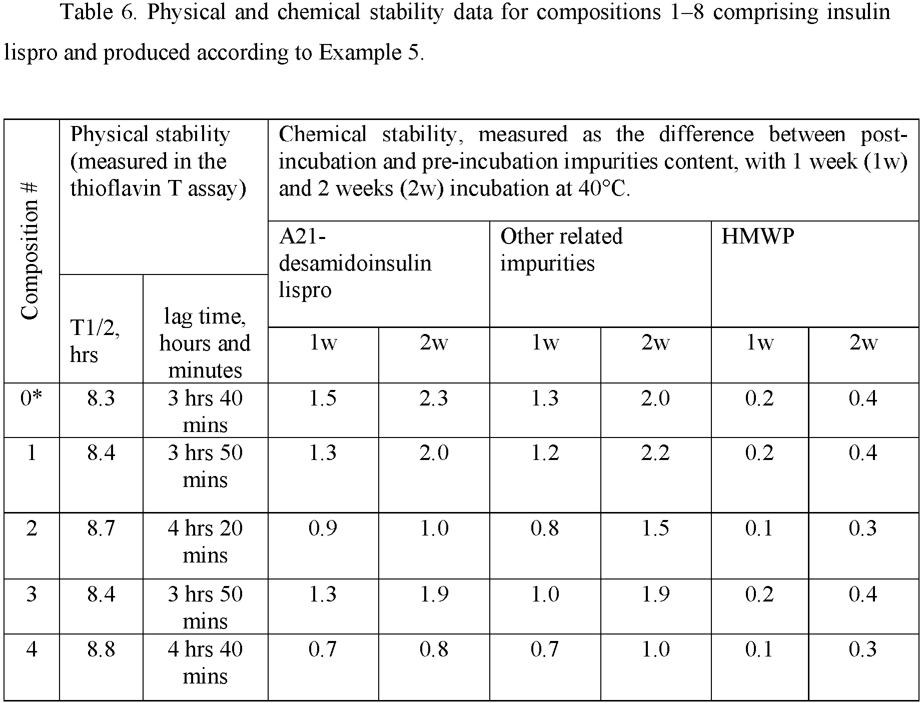

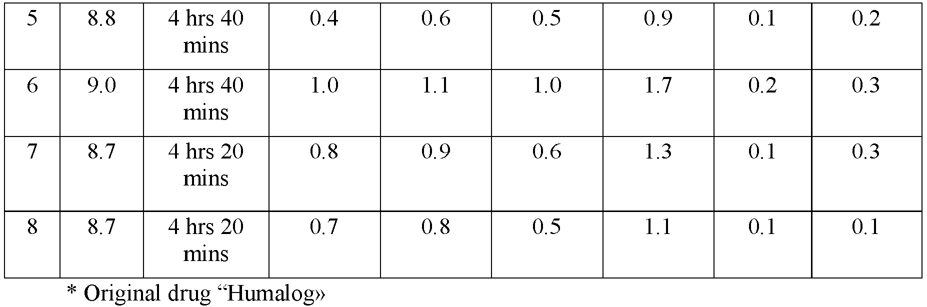

### References:

1. O. M. Selivanova, S. Yu. Grishin, A. V. Glyakina, et al. Analysis of insulin analogues and strategy for their further development. Advances in biological chemistry. 2018, 58: 313-346.
2. Brange J., Owens D. R., Kang S., Volund A. Monomeric Insulins and Their Experimental and Clinical Implications. Diabetes Care. 1990, 13(9): 923-954. doi:10.2337/diacare.13.9.923
3. Schlein M. Insulin Formulation Characterization - the Thioflavin T Assays. AAPS Journal. 2017, 19(2): 397 - 408.
4. Directions for medical use of Fiasp DP. URL: http://grls.rosminzdrav.ru (accessed on 30 Aug 2022).
5. Brange J. Stability of Insulin. Kluwer Academic Publisher. 1994, 18-23.
6. Paquot N., Scheen A. J. Faster aspart insulin (FIASP®). Revue Medicale de Liege. 2018, 73 (4): 211-215.
7. Muchmore D. B. Pump Users Clamor for Faster Insulin: Is Fast-Acting Insulin Aspart Ready for Them? Journal of Diabetes Science and Technology. 2018, 12(1): 152-154. doi:10.1177/1932296817750166
8. Ahem T. J., Manning M. C. Stability of Protein Pharmaceuticals. Plenum Press. New York. 1992.

## Claims

1. A composition for reducing blood glucose level in a subject, wherein the composition comprises a fast-acting insulin and an amino acid, wherein the amino acid is lysine or a salt thereof, or a combination of lysine or a salt thereof with arginine or a salt thereof.

2. The composition of claim 1, wherein the fast-acting insulin is selected from the group consisting of insulin aspart (B28Asp), insulin lispro (B28LysB29Pro), and insulin glulisine (3BLys29BGlu).

3. The composition of claim 2, wherein fast-acting insulin is insulin aspart (B28Asp).

4. The composition of claim1, wherein the amino acid is lysine or a salt thereof.

5. The composition of claim 1, wherein the amino acid is a combination of lysine or a salt thereof with arginine or a salt thereof.

6. The composition of claim 3, wherein the amino acid is lysine or a salt thereof.

7. The composition of claim 3, wherein the amino acid is a combination of lysine and arginine.

8. The composition of claim 2, wherein the fast-acting insulin is insulin glulisine (3BLys29BGlu).

9. The composition of claim 8, wherein the composition further comprises methionine.

10. The composition of claim 1, wherein the insulin is present in an amount ranging from about 0.2 mM (33 U/ml) to about 2 mM (333 U/ml).

11. The composition of claim 1, wherein the insulin is present in an amount ranging from about 0,3 mM (50 U/ml) to about 1,2 mM (200 U/ml).

12. The composition of claim 10 or claim 11, wherein the insulin is insulin aspart and the composition further comprises a nicotinic acid compound.

13. The composition of claim 12, wherein the composition comprises nicotinamide as the nicotinic acid compound.

14. The composition of claim13, wherein the composition comprises from about 1 mM to about 200 mM of nicotinamide.

15. The composition of claim 10 or 11, wherein the composition comprises from about 1 mM to about 100 mM of lysine or a salt thereof.

16. The composition of claim 10 or 11, wherein the composition comprises from about 1 mM to about 100 mM of a combination of lysine or a salt thereof with arginine or a salt thereof.

17. The composition of claim 1, wherein the composition further comprises a metal ion, preserving agent(s), isotonic agent(s), stabilizer(s), buffer(s), and acidity regulators.

18. The composition of claim 1 for use in the treatment or prevention of hyperglycemia, type 2 diabetes mellitus, impaired glucose tolerance, type 1 diabetes mellitus.

19. An injectable insulin preparation for reducing blood glucose level in a subject, said preparation comprising the composition according to any one of claims 1-17.
